# EUROPEAN PATENT SPECIFICATION

(11) **EP 1 595 149 B1**
(45) Date of publication and mention of the grant of the patent: **20.06.2007**
(21) Application number: 04705878.9
(22) Date of filing: 28.01.2004
(51) Int. Cl.: G01P 15/00, A61B 5/00, A61B 5/22

(54) **DEVICE FOR DETERMINING A VALUE THAT IS REPRESENTATIVE OF ACCELERATIONS AS WELL AS AN ERGOMETER**
VORRICHTUNG ZUR BESTIMMUNG EINES BESCHLEUNIGUNGSABHÄNGIGEN WERTES UND ERGOMETER
ERGOMETRE ET DISPOSITIF PERMETTANT DE DETERMINER UNE VALEUR QUI EST REPRESENTATIVE D'ACCELERATIONS

(30) Priority: 07.02.2003 EP 03100256
(43) Date of publication of application: 16.11.2005
(73) Proprietor: Koninklijke Philips Electronics N.V., 5621 BA Eindhoven (NL)
(72) Inventor: MIMNAGH-KELLEHER, Gillian, A., NL-5656 AA Eindhoven (NL); VERSPAGET, Henricus, A., NL-5656 AA Eindhoven (NL); BREMER, Joannes, G., NL-5656 AA Eindhoven (NL); ROMMERS, Adrianus, P., J., M., NL-5656 AA Eindhoven (NL); VERHOEVEN, Wilhelmus, L., M., C., NL-5656 AA Eindhoven (NL)
(74) Representative: Rolfes, Johannes Gerardus Albertus
(86) International application number: PCT/IB2004/050056
(87) International publication number: WO 2004/070392

(56) References cited:
- EP-A- 1 256 316
- WO-A-81/01507
- US-A- 4 192 000
- US-A- 5 272 476
- US-A1- 2002 008 630
- BOUTEN C V C ET AL: "EFFECTS OF PLACEMENT AND ORIENTATION OF BODY-MIXED ACCELEROMETERS ON THE ASSESSMENT OF ENERGY EXPENDITURE DURING WALKING" MEDICAL AND BIOLOGICAL ENGINEERING AND COMPUTING, PETER PEREGRINUS LTD. STEVENAGE, GB, vol. 35, no. 1, 1997, pages 50-56, XP000643309 ISSN: 0140-0118
- BOUTEN C V C ET AL: "A TRIAXIAL ACCELEROMETER AND PORTABLE DATA PROCESSING UNIT FOR THE ASSESSMENT OF DAILY PHYSICAL ACTIVITY" IEEE TRANSACTIONS ON BIOMEDICAL ENGINEERING, IEEE INC. NEW YORK, US, vol. 44, no. 3, 1 March 1997 (1997-03-01), pages 136-147, XP000658916 ISSN: 0018-9294
- PATENT ABSTRACTS OF JAPAN vol. 2003, no. 02, 5 February 2003 (2003-02-05) & JP 2002 296294 A (MITSUMI ELECTRIC CO LTD), 9 October 2002 (2002-10-09)

## Description

The invention relates to a device for determining a value that is representative of accelerations in at least two directions perpendicular to each other, the device comprising a sensor system with which the acceleration in each of the mutually perpendicular directions can be converted into an electric signal while the value can be determined from the electric signals by signal processing means.

The invention further relates to an ergometer for measuring a value that is representative of a physical effort of an individual, which ergometer comprises an arrangement that includes a sensor system with which the acceleration in each of the mutually perpendicular directions can be converted into electric signals while the value can be determined from the electric signals by signal processing means.

Such a device known from US-B1-6,361,508 comprises a sensor that is suitable for measuring values that are representative of accelerations in three mutually perpendicular directions of a moving individual. The sensor generates three electric signals which are linearly proportional to the accelerations in the individual directions. The device further comprises signal processing means which include three circuits. The three separate electric signals are individually processed by the three circuits.

A disadvantage of this device is that the device is relatively complex and expensive as a result of the three circuits.

It is an object of the present invention to provide a device with a relatively simple and cost-effective structure by means of which a value that is representative of the accelerations can be obtained.

With the device according to the invention this object is achieved in that the electric signals can be added together by means of an adding element preceding the signal processing means.

By adding the electric signals together prior to the signal processing means, only a single electric circuit then needs to be used for determining the value. This provides a device with a simple structure that can furthermore be manufactured in a compact and cost-effective way.

In W081/01507 an ergometer is disclosed comprising an accelerometer.

EP-1.256.316 describes a portable device having means for calculating the Physical Activity Index based on the signal generated by an acceleration sensor.

XP000643309, "Effects of placement and orientation of body-fixed accelerometers on the assessment of energy expenditure during walking" from Bouten et al., discloses test results for determining the best location of a accelerometer on a body.

An embodiment of the device according to the invention is characterized in that in the adding element the connections conducting the electric signals are connected in parallel.

In such manner the signals are added together in a simple and compact manner by means of the connections already present.

Another embodiment of the device according to the invention is characterized in that the sensor system comprises at least a sensor that includes a flexible strip made of piezoelectric material.

Such a sensor can be manufactured cost effectively while with the aid of the sensor relatively accurate accelerations in and contrary to a direction perpendicular to the strip can be measured.

The invention also aims at providing an ergometer that has a relatively simple and cost-effective structure.

This object is achieved according to the invention in that prior to the signal processing means the electric signals can be added together by means of an adding element.

The value to be determined by the signal processing means from the added-together electric signals is representative of the accelerations carried out by an individual such as a human being and is thus representative of the efforts made by the individual. With the aid of the ergometer according to the invention a value that is representative of the efforts can thus be determined in a simple manner.

An embodiment of the ergometer according to the invention is characterized in that the ergometer comprises a database in which the value is correlated to an energy value such as, for example, a nutritional value.

Such an embodiment is advantageous in that the value that is representative of the efforts of an individual can be coupled to an energy value such as, for example, a nutritional value to determine, for example, the number of kilojoules a certain effort has cost.

A further embodiment of the ergometer according to the invention is characterized in that the ergometer comprises a memory in which the energy values can be stored over a certain period of time.

In this way it is possible to compare values that are representative of individual efforts over time.

Another embodiment of the ergometer according to the invention is characterized in that the ergometer comprises a screen on which the instantaneous efforts and/or average effort over a certain period can be displayed in energy values.

Such an embodiment is advantageous in that the user can directly read the instantaneous effort and/or average effort.

Yet another embodiment of the ergometer according to the invention is characterized in that the ergometer comprises a coupling to which a computer can be connected for transferring stored data from the ergometer to the computer.

In this way the data can be transferred to a computer in which more storage space is present. In addition, the data can be processed more extensively in the computer and it is possible to make graphs of the effort recorded over time by the ergometer.

These and other aspects of the invention are apparent from and will be elucidated with reference to the embodiments described hereinafter.

In the drawings:
Fig. 1 shows a schematic view of a device according to the invention,
Fig. 2 shows a plan view of a sensor system of the device according to the invention shown in Fig. 1.

Like elements in the Figures have like references.

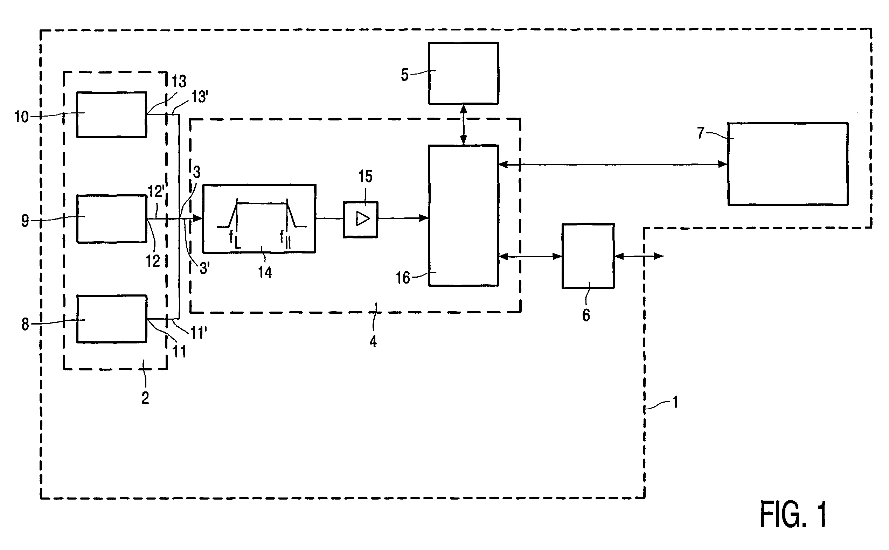

Fig. 1 shows a device according to the invention comprising a sensor system 2 which is connected to an adding element 3, signal processing means 4 connected to the adding element 3 and a memory 5 connected to the signal processing means 4, connection 6 and display means 7.

The sensor system 2 comprises three sensors 8, 9, 10 which have each an output 11, 12, 13. The outputs 11, 12, 13 of the sensors 8, 9, 10 are connected to the adding element 3 via parallel arranged electroconductive connections 11', 12', 13', which adding element 3 is connected to the signal processing means 4 via a single electrical connection 3'. The signal processing means 4 comprise a filter arrangement 14, an amplifier 15 and a processor 16.

By means of the processor 16 a value is determined and possibly stored in a memory 19. A computer (not shown) is connected, for example serially connected, to the connection 6. The display means 7 such as a screen can display the values determined by means of the processor 16.

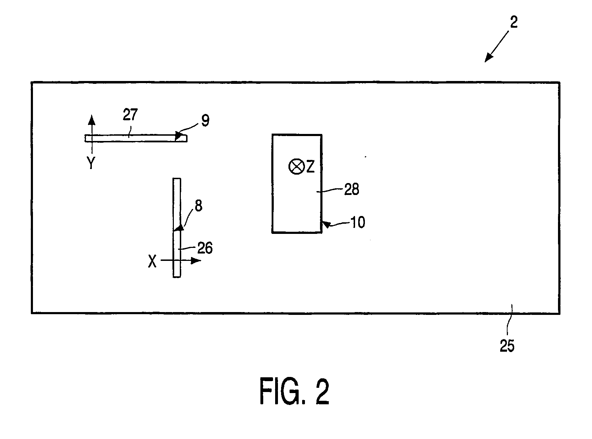

Fig. 2 shows the sensor system 2 of the device 1 according to the present invention. The sensor system 2 comprises a frame on which three sensors working independently 8, 9, 10 are installed.

The sensors 8, 9, 10 each comprise a piezoelectric strip 26, 27, 28 embedded in recesses in the frame 25, the strips 26, 27, 28 being retained at one end by means of a supporting agent (not shown). The strips 26, 27 extend through the frame 25, the strip 26 being oriented such that the acceleration in the X direction is recorded by means of the strip 26 and strip 27 being oriented such that the acceleration in the Y direction is recorded by means of the strip 27. The strip 28 is embedded in parallel with the frame 25 in the recess so that the acceleration in the Z direction is recorded by means of the strip 28.

The device, for example, attached to a belt is carried by a person. If this person walks or runs, forces will be exerted on the strips 26, 27, 28 as a result of the attendant accelerations, which forces lead to the strips 26, 27, 28 being bent. The bending will generate an electrical load which is proportional to the acceleration. The generated currents I₈, I₉, I₁₀ are transferred to the outputs 11, 12, 13 of the sensors 8, 9, 10.

The currents I₈, I₉, I₁₀ generated by means of the sensors 8, 9, 10 are representative of the accelerations in the X, Y, Z directions. The currents I₈, I₉, I₁₀ of the sensors 8, 9, 10 are led to an adding element 3 through the outputs 11, 12 and 13 and the connections 11', 12', 13'. In the adding element 3 the currents I₈, I₉, I₁₀ are added together, which produces a total current: Iₜₒₜ = I₈ + I₉ + I₁₀.

After the adding element 3 the total current Iₜₒₜ is processed to a value that can be processed by the processor 16. The value can be converted in the processor 16 into a magnitude that indicates the degree of effort. The values are correlated with energy values such as, for example, nutritional values. This is possible by means of a database stored in the processor 16 in which base each value has an associated energy value.

By means of the memory 19 it is, for example, possible to save the results for a certain period of time. The results can also be written in a computer via connection 6 with which computer the data produced by the device 1 can be processed still further.

The sensor system 2 may also have two sensors for recording, for example, only the acceleration in the X and Y directions.

## Claims

1. A device for determining a value that is representative of accelerations in at least two directions perpendicular to each other, the device comprising at least two sensors, wherein with each sensor the acceleration in one of the mutually perpendicular directions is convertable into an electric signal while the value is determinable from the electric signals of said sensors by signal processing means, **characterized in that** prior to the signal processing means the electric signals in the form of a current of each sensor are addable together by means of an adding element to a total current, wherein the signal processing means are arranged to convert said total current to the value that is representative of accelerations.

2. A device as claimed in claim 1, **characterized in that** in the adding element the connections conducting the electric signals are arranged in parallel.

3. A device as claimed in claim 1 or 2, **characterized in that** a sensor system comprises at least a sensor which comprises a flexible strip made of piezoelectric material.

4. A device as claimed in any one of the preceding claims 1-3, **characterized in that** the signal processing means comprise a signal amplifier, a bandpass filter and a processor.

5. An ergometer for measuring a value that is representative of accelerations in at least two directions perpendicular to each other which value is convertible with signal processing means into a magnitude that indicates the degree of effort, the ergometer comprising a device that includes at least two sensors, wherein with each sensor the acceleration in one of the mutually perpendicular directions is convertable into an electric signal while the value is determinable from the electric signals of said sensors by signal processing means, **characterized in that** prior to the signal processing means the electric signals in the form of a current of each sensor are addable together by means of an adding element to a total current, wherein the signal processing means are arranged to convert said total current to the value that is representative of accelerations.

6. An ergometer as claimed in claim 5, **characterized in that** in the adding element the connections conducting the electric signals are arranged in parallel.

7. Ergometer as claimed in claim 5 or 6, **characterized in that** the ergometer comprises a database in which the value is correlated to an energy value such as, for example a nutritional value.

8. Ergometer as claimed in claim 7, **characterized in that** the ergometer comprises a memory in which energy values can be stored over a certain period of time.

9. Ergometer as claimed in claim 7 or 8, **characterized in that** the ergometer comprises a screen on which the instantaneous effort and/or average effort can be displayed in energy values over a certain period.

10. An ergometer as claimed in any one of the preceding claims 5-8, **characterized in that** the ergometer comprises a coupling to which a computer can be connected, for transferring stored data from the ergometer to the computer.

11. An ergometer as claimed in any one of the preceding claims 5-10, **characterized in that** the sensor system comprises at least a sensor that includes a flexible strip made of piezoelectric material.

12. An ergometer as claimed in one of the preceding claims 5-11, **characterized in that** the signal processing means comprise a signal amplifier, a bandpass filter and a processor.

## Patentansprüche

1. Einrichtung zum Ermitteln eines Wertes, der repräsentativ für Beschleunigungen in zumindest zwei senkrecht zueinander stehenden Richtungen ist, welche Einrichtung zumindest zwei Sensoren umfasst, wobei mit jedem Sensor die Beschleunigung in einer der zueinander senkrechten Richtungen in ein elektrisches Signal umgewandelt werden kann, wobei der Wert mittels Signalverarbeitungsmitteln aus den elektrischen Signalen der genannten Sensoren ermittelt werden kann, **dadurch gekennzeichnet, dass** vor den Signalverarbeitungsmitteln die elektrischen Signale in Form eines Stroms jedes Sensors mit Hilfe eines Addierelementes miteinander zu einem Gesamtstrom addiert werden können, wobei die Signalverarbeitungsmittel ausgebildet sind, den genannten Gesamtstrom in den Wert umzuwandeln, der repräsentativ für Beschleunigungen ist.

2. Einrichtung nach Anspruch 1, **dadurch gekennzeichnet, dass** in dem Addierelement die Verbindungen, die die elektrischen Signale leiten, parallel geschaltet sind.

3. Einrichtung nach Anspruch 1 oder 2, **dadurch gekennzeichnet, dass** ein Sensorsystem zumindest einen Sensor umfasst, der einen aus piezoelektrische Material hergestellten flexiblen Streifen umfasst.

4. Einrichtung nach einem der vorhergehenden Ansprüche 1-3, **dadurch gekennzeichnet, dass** die Signalverarbeitungsmittel einen Signalverstärker, ein Bandpassfilter und einen Prozessor umfassen.

5. Ergometer zum Messen eines Wertes, der repräsentativ für Beschleunigungen in zumindest zwei senkrecht zueinander stehenden Richtungen ist, welcher Wert mittels Signalverarbeitungsmitteln in eine Größe umgewandelt werden kann, die den Grad einer Anstrengung anzeigt, wobei das Ergometer eine Einrichtung umfasst, die zumindest zwei Sensoren enthält, wobei mit jedem Sensor die Beschleunigung in einer der zueinander senkrechten Richtungen in ein elektrisches Signal umgewandelt werden kann, wobei der Wert mittels Signalverarbeitungsmitteln aus den elektrischen Signalen der genannten Sensoren ermittelt werden kann, **dadurch gekennzeichnet, dass** vor den Signalverarbeitungsmitteln die elektrischen Signale in Form eines Stroms jedes Sensors mit Hilfe eines Addierelementes miteinander zu einem Gesamtstrom addiert werden können, wobei die Signalverarbeitungsmittel ausgebildet sind, den genannten Gesamtstrom in den Wert umzuwandeln, der repräsentativ für Beschleunigungen ist.

6. Ergometer nach Anspruch 5, **dadurch gekennzeichnet, dass** in dem Addierelement die Verbindungen, die die elektrischen Signale leiten, parallel geschaltet sind.

7. Ergometer nach Anspruch 5 oder 6, **dadurch gekennzeichnet, dass** das Ergometer eine Datenbasis umfasst, in der der Wert mit einem Energiewert korreliert ist, wie z.B. einem Nährwert.

8. Ergometer nach Anspruch 7, **dadurch gekennzeichnet, dass** das Ergometer einen Speicher umfasst, in dem Energiewerte für eine gewisse Zeitdauer gespeichert werden können.

9. Ergometer nach Anspruch 7 oder 8, **dadurch gekennzeichnet, dass** das Ergometer einen Bildschirm umfasst, auf dem die momentane Anstrengung und/oder mittlere Anstrengung über einen gewissen Zeitraum in Energiewerten wiedergegeben werden kann.

10. Ergometer nach einem der vorhergehenden Ansprüche 5-8, **dadurch gekennzeichnet, dass** das Ergometer eine Kopplung umfasst, mit der ein Computer verbunden werden kann, um gespeicherte Daten von dem Ergometer zu dem Computer zu übertragen.

11. Ergometer nach einem der vorhergehenden Ansprüche 5-10, **dadurch gekennzeichnet, dass** das Sensorsystem zumindest einen Sensor umfasst, der einen aus piezoelektrischem Material hergestellten flexiblen Streifen enthält.

12. Ergometer nach einem der vorhergehenden Ansprüche 5-11, **dadurch gekennzeichnet, dass** die Signalverarbeitungsmittel einen Signalverstärker, ein Bandpassfilter und einen Prozessor umfassen.

## Revendications

1. Dispositif permettant de déterminer une valeur qui est représentative pour les accélérations se produisant dans au moins deux directions perpendiculaires entre elles, le dispositif comprenant au moins deux détecteurs, dans lequel chaque détecteur permet de convertir l'accélération se produisant dans l'une des directions mutuellement perpendiculaires en un signal électrique, alors que la valeur peut être déterminée à partir des signaux électriques desdits détecteurs par des moyens de traitement de signal, **caractérisé en ce qu'**avant le moyen de traitement de signal, les signaux électriques sous forme d'un courant de chaque détecteur peuvent être additionnés ensemble à l'aide d'un élément d'addition en un courant total, dans lequel les moyens de traitement de signal sont disposés pour convertir ledit courant total en la valeur qui est représentative pour les accélérations.

2. Dispositif selon la revendication 1, **caractérisé en ce que** dans l'élément d'addition les connexions assurant la conduction des signaux électriques sont disposées en parallèle.

3. Dispositif selon la revendication 1 ou 2, **caractérisé en ce qu'**un système de détection comprend au moins un détecteur qui comprend une bande flexible réalisée en un matériau piézo-électrique.

4. Dispositif selon l'une des revendications précédentes 1 à 3, **caractérisé en ce que** les moyens de traitement de signal comprennent un amplificateur de signal, un filtre passe-bande et un processeur.

5. Ergomètre pour la mesure d'une valeur qui est représentative pour les accélérations se produisant dans au moins deux directions perpendiculaires entre elles, laquelle valeur peut être convertie à l'aide de moyens de traitement de signal en une grandeur qui indique le degré d'effort, l'ergomètre comprenant un dispositif qui est muni d'au moins deux détecteurs, dans lequel chaque détecteur permet de convertir l'accélération se produisant dans l'une des directions mutuellement perpendiculaires en un signal électrique, alors que la valeur peut être déterminée à partir des signaux électriques desdits détecteurs par des moyens de traitement de signal, **caractérisé en ce qu'**avant les moyens de traitement de signal, les signaux électriques sous forme d'un courant de chaque détecteur sont additionnés ensemble à l'aide d'un élément d'addition en un courant total, dans lequel les moyens de traitement sont disposés pour convertir ledit courant total en la valeur qui est représentative pour les accélérations.

6. Ergomètre selon la revendication 5, **caractérisé en ce que** dans l'élément d'addition les connexions assurant la conduction des signaux électriques sont disposées en parallèle.

7. Ergomètre selon la revendication 5 ou 6, **caractérisé en ce que** l'ergomètre comprend une base de données dans laquelle la valeur est corrélée à une valeur d'énergie, telle que, par exemple, une valeur nutritionnelle.

8. Ergomètre selon la revendication 7, **caractérisé en ce que** l'ergomètre est muni d'une mémoire dans laquelle peuvent être stockées les valeurs d'énergie sur une période de temps déterminée.

9. Ergomètre selon la revendication 7 ou 8, **caractérisé en ce que** l'ergomètre est muni d'un écran sur lequel peu(ven)t être reproduit(s) l'effort instantané et/ou l'effort moyen en valeurs d'énergie sur une période déterminée.

10. Ergomètre selon l'une des revendications précédentes 5 à 8, **caractérisé en ce que** l'ergomètre comprend un couplage auquel peut être connecté un ordinateur, par exemple pour transférer les données stockées à partir de l'ergomètre à l'ordinateur.

11. Ergomètre selon l'une des revendications précédentes 5 à 10, **caractérisé en ce que** le système de détection est muni d'au moins un détecteur qui comprend une bande flexible réalisée en un matériau piézo-électrique.

12. Ergomètre selon l'une des revendications précédentes 5 à 11, **caractérisé en ce que** les moyens de traitement de signal sont munis d'un amplificateur, d'un filtre passe-bande et d'un processeur.
